# EUROPEAN PATENT SPECIFICATION

(11) **EP 3 672 423 B1**
(45) Date of publication and mention of the grant of the patent: **24.03.2021**
(21) Application number: 19790544.1
(22) Date of filing: 24.10.2019
(51) Int. Cl.: A23L 2/395, A23L 33/15, A23P 10/22, A23P 10/40, A23P 30/20

(54) **EXTRUDATES COMPRISING VITAMIN B9**
VITAMIN B9 ENTHALTENDE EXTRUDATE
EXTRUDATS COMPRENANT DE LA VITAMINE B9

(30) Priority: 30.10.2018 EP 18203304
(43) Date of publication of application: 01.07.2020
(73) Proprietor: DSM IP Assets B.V., 6411 TE Heerlen (NL)
(72) Inventor: CONNOLLY, Alan, 4303 Kaiseraugst (CH); RIEGER, Henry, 4303 Kaiseraugst (CH); SPREITER, Simon, 4303 Kaiseraugst (CH)
(74) Representative: Kuhn, Dieter
(86) International application number: PCT/EP2019/079050
(87) International publication number: WO 2020/089032

(56) References cited:
- WO-A1-2005/053433
- WO-A1-2014/083065
- WO-A1-2015/171906
- WO-A2-02/05667
- GB-A- 2 281 841

## Description

### Technical field

The present invention relates to water-dispersible, edible particles that contain vitamins. Such particles may be used to prepare food or beverages.

### Background of the invention

Folic acid has been known for a long time. Folic acid is also referred to as vitamin B9.

Folic acid is commercially available. EP 1 026 166 B1 discloses a process for preparing folic acid.

Folic acid is water-soluble. Example 1 of EP 0 954 986 B1 discloses a beverage that contains folic acid.

Enriched beverages can be produced by dispersing an instant powder in water, fruit juice or alike. Before usage, such instant powders are stored in a bag. To be commercially valuable, the closed bag with its content should have a long the shelf-life.

Different methods to manufacture instant powders are known. A preferred method is extrusion. Unfortunately, vitamin extrudates which contain folic acid have a short or even very short shelf-life. In the presence of riboflavin and/or thiamin, shelf-life of extrudates which contain folic acid is even shorter.

Shelf-life can be improved by choosing a different manufacturing method such as spray-drying. However, these alternative manufacturing methods trigger high capital expense (CAPEX) and have other disadvantages such as the need for organic solvents and/or low output per working hour.

There is a need for a water-dispersible, edible powder that contains vitamin B9 and that is storage-stable even if riboflavin and/or thiamin are also present.

The manufacturing of such powder should be low-cost, easy and/or solvent-free

WO 2005/053433 A1 discloses a process for the preparation of enriched reconstituted rice which comprises the steps of (a) hydrating a mixture of a comminuted rice matrix material, at least one micronutrient, and an emulsifier to obtain a paste containing 15-35 wt.-% of water and kneading the paste obtained while heating to about 70-100°C for no more than about 5 minutes until the rice starch is semi-gelatinized; (b) forming the semi-gelatinized mass to strands and cutting them to obtain grains similar or equal to the size of rice grains; and (c) drying the grains to a moisture content of no more than 15 wt.-%.

### Summary of the invention

The problems underlying the present invention are solved by extruding a composition which comprises vitamin B9, at least one pH buffer, at least one filler, optionally at least one lubricant and water.

The addition of a pH buffer significantly reduces the decomposition of vitamin B9 during storage of the extrudates. Surprisingly, the decomposition of vitamin B9 is reduced even if other members of the B vitamin group are present. Usually, folic acid decomposes quickly in the presence of riboflavin and/or thiamin.

A *solid* pH buffer can be used to reduce the decomposition of the extrudates' vitamin B9. This is surprising as only little water is used during the extrusion process. If the composition to be extruded contained a lot of water, extensive drying of the obtained extrudates would be needed. Extensive drying increases energy cost and reduces the output per working hour.

Solid-state reactions are known to be notoriously slow. It is surprising that a solid pH buffer can be used in a relatively fast process using a short extruder and little water only.

### Detailed description of the invention

The present invention relates to an extruded strand. After extrusion, the obtained strand is cut into smaller pieces which are then dried. The thus obtained plurality of extrudates is a water-soluble or water-dispersible coarse powder. To increase the flowability of the powder, the extrudates may be spheronized before drying.

### Extruded strand

The extruded strand of the invention is obtained by extruding a composition which comprises vitamin B9, at least one pH buffer, at least one filler, optionally at least one lubricant and water.

In the context of the present invention, vitamin B9 is a synonym of folic acid. In one embodiment of the invention, the term "folic acid" includes congeners of folic acid such as tetrahydrofolic acid, methyltetrahydrofolate, methenyltetrahydrofolate, folinic acid, folate, and folacin.

The extruded strand of the composition comprises preferably at least one further micronutrient. Preferably, said at least one further micronutrient is selected from the group consisting of minerals, fat-soluble vitamins, water-soluble vitamins, amino acids, polyunsaturated fatty acids and carotenoids. Water-soluble vitamins according to the present invention are preferably vitamin B1 (thiamine), vitamin B2 (riboflavin), vitamin B3 (niacin, niacinamide), vitamin B5 (pantothenic acid), vitamin B6 (pyridoxine, pyridoxamine, pyridoxal), vitamin B7 (biotin), vitamin B9 and vitamin B12 (cyanocobalamin, hydroxycobalamin, methylcobalamin).

In a preferred embodiment of the invention, the extruded strand of the invention is obtained by extruding a composition which comprises vitamin B9, vitamin B1, vitamin B2, at least one pH buffer, at least one filler, optionally at least one lubricant and water.

In the context of the present invention, a "pH buffer" is a mixture of a weak acid and a salt of its conjugate base, or a mixture of a weak base and a salt of its conjugate acid. In a preferred embodiment of the invention, the pH buffer is a mixture of a weak acid and a salt of its conjugate base wherein both, the weak acid and the salt of its conjugate base, are solid at room temperature and at standard atmosphere. "Buffer" means that the pH of an aqueous solution comprising a suitable amount of a pH buffer changes very little when a small amount of strong acid or base is added. In the context of the present invention, non-edible pH buffers are excluded. In the context of the present invention, "edible" refers to a composition having food grade or feed grade. A composition having food grade is meant for human consumption. Also excluded are pH buffers which do not reduce the decomposition of the extruded strand's vitamin B9 when stored over 3 months in a closed aluminium bag at 30°C and 65% humidity. The pH buffer of the invention is preferably a mixture of (i) ascorbic acid and (ii) an edible salt of ascorbic acid such as sodium ascorbate. Less preferred pH buffers are
- a mixture of citric acid and an edible citrate salt;
- a mixture of carbonic acid and an edible carbonate salt

In a preferred embodiment of the invention, the extruded strand of the invention is obtained by extruding a composition which comprises vitamin B9, at least one pH buffer, at least one filler, at least one lubricant and water, wherein said at least one pH buffer is a mixture of (i) ascorbic acid and (ii) an edible salt of ascorbic acid. In an even more preferred embodiment of the invention, the extruded strand of the invention is obtained by extruding a composition which comprises vitamin B9, vitamin B1, vitamin B2, at least one pH buffer, at least one filler, at least one lubricant and water, wherein said at least one pH buffer is a mixture of (i) ascorbic acid and (ii) an edible salt of ascorbic acid.

The extruded strand comprises at least one "filler". In a preferred embodiment of the invention, the filler is (i) edible and (ii) water-soluble or water-dispersible. The extruded strand comprises preferably at least 50 weight-%, more preferably at least 60 weight-% and most preferably at least 70 weight-% of at least one filler, based on the total weight of the extruded strand and not including water. Preferred fillers are starch (such as wheat starch) and semolina (such as durum wheat semolina). Preferably, a mixture of starch and semolina is used as filler.

Thus, the extruded strand is preferably obtained by extruding a composition comprising vitamin B9, at least one pH buffer, at least one lubricant, water and at least 50 weight-% of at least one filler, based on the total weight of the extruded strand and not including water, wherein said at least one filler is preferably a mixture of starch and semolina such as a mixture of wheat starch and durum wheat semolina. In an even more preferred embodiment of the invention, the extruded strand is preferably obtained by extruding a composition comprising vitamin B9, vitamin B1, vitamin B2, at least one pH buffer, water, at least one lubricant and at least 50 weight-% of at least one filler, based on the total weight of the extruded strand and not including water, wherein said at least one filler is preferably a mixture of starch and semolina such as a mixture of wheat starch and durum wheat semolina.

Preferably, the extruded strand comprises at least one lubricant. Preferably, said at least one lubricant is liquid at room temperature. Preferred lubricants are edible oils such as medium chain triglyceride (MCT), corn oil, vegetable oil and coconut oil. MCT is the most preferred lubricant. Thus, in the most preferred embodiment of the invention, the extruded strand of the invention is obtained by extruding a composition which comprises vitamin B9, vitamin B1, vitamin B2, at least one pH buffer, at least one filler, at least one lubricant such as MCT and water, wherein said at least one pH buffer is a mixture of (i) ascorbic acid and (ii) an edible salt of ascorbic acid.

The diameter of the extruded strand depends on the die of the extruder. The extruder's die has at least one hole. In most cases, extruder's die has multiple holes, wherein each hole has the same diameter. The diameter of the extruder's hole(s) is preferably from 0.1 mm to 2 mm, more preferably from 0.3 mm to 1.2 mm and most preferably from 0.4 mm to 0.8 mm.

Shaping a composition into a strand is only possible if the composition to be shaped has a certain plasticity. According to the present invention, the plasticity the composition to be extruded can be adapted by the amount of water. The extruded strand comprises preferably from 15 weight-% to 60 weight-% water, more preferably from 20 weight-% to 55 weight-% water and most preferably from 25 weight-% to 45 weight-% water, based on the total weight of the extruded strand.

The plasticity the composition to be extruded can also be adapted by increasing the extrusion temperature. The temperature at the die of the extruder is preferably from 50°C to 120°C, more preferably from 55°C to 90°C and most preferably from 60°C to 80°C. Temperatures of more than 120°C are not preferred as such high temperature may have a negative impact on the vitamins.

The length of the extruded strand is variable. In a continuous extrusion process, the strand does not have a well-defined length. Furthermore, the length of the extruded strand depends on the cutting process. Typically, the strand is cut at the die face. Instead of die-face cutting, downstream cutting can also be applied.

### Plurality of extrudates

A plurality is more than one. Thus, the expression "plurality of extrudates" refers to two or more extrudates. Preferably, the plurality of extrudates of the invention is a powder having a mass between 1 g and 10 kg.

The plurality of extrudates of the invention are preferably water-soluble or water-dispersible. Therefore, the extrudates are preferably essentially free of fat, essentially free of fat-soluble proteins and/or essentially free of organic solvent. In this context, the expression "essentially free of" means less than 1 weight-%, preferably less than less than 0.8 weight-%, more preferably less than less than 0.5 weight-% and most preferably less than less than 0.2 weight-%, based on the total weight of the plurality of extrudates. In the context of the present invention, the term "fat" does not include liquids such as a liquid lubricant or any other oil.

The plurality of extrudates of the invention are obtainable by cutting the herein described extruded strand. Thus, the size of the individual extrudates is preferably determined the diameter of the extruder's hole(s). In a less preferred embodiment of the invention, processing conditions as chosen such that the extrudate expands upon exiting the die. The size of the individual extrudates is also determined by the cutting process. Preferably, cutting is done in such manner that the length of the extrudates corresponds approximately to the diameter of the extruder's hole(s).

After cutting, the shape of the individual extrudates is cylinder-like. To increase flowability of the plurality of extrudates, the still wet extrudates may optionally be shaped in a spheronizer.

Preferably, the extrudates are at least partially dried after cutting and after optional spheronization. For drying, a fluid bed drier or any other suitable apparatus may be used. Typically, drying is done for 30 minutes to 90 minutes at a temperature between 40 °C and 80 °C. After drying the extrudates may still comprise some residual water. The extrudates of the plurality of extrudates comprise preferably less than 10 weight-%, more preferably less than 8 weight-% and most preferably less than 5 weight-% water, based on the total weight of the extrudate.

The extrudates of the plurality of extrudates comprise at least one pH buffer. Thus, a certain pH is reached if a predetermined amount of extrudates are solved or dispersed in deionised water. The pH of a liquid obtainable by dispersing 1 g of the plurality of extrudates in 40 ml deionised water is at room temperature (i.e. 23°C) preferably from 4.6 to 6, more preferably from 4.65 to 5.2 and most preferably from 4.7 to 5. Preferably, said 1 g is measured after having applied the drying step.

Without wishing to be bound by theory, it is believed that the above indicated pH ranges reduce decomposition of vitamin B9, vitamin B1 and vitamin B2 during storage in an extrudate which comprises said three B vitamins. Said pH ranges can be achieved by a mixture of (i) ascorbic acid and (ii) an edible salt of ascorbic acid. However, said pH ranges can also be achieved with other edible pH buffers such as pH buffers based on citric acid or based on calcium carbonate. pH buffers which are not suitable to reach the above indicated pH ranges are excluded.

The amount and type of pH buffer is chosen such that storage stability is good even if riboflavin and/or thiamin are also present.

The vitamin B9 content of the extrudates of the plurality of extrudates is after 3 months storage in a closed aluminium bag at 30°C and 65% humidity preferably at least 70%, more preferably at least 75% and most preferably at least 80% of the vitamin B9 content of said extrudates before storage.

### Use of pH buffer

The present invention also relates to the use of the pH buffer to increase the shelf-life of an extrudate which comprises vitamin B9. In a preferred embodiment, the invention relates to the use of a pH buffer to increase the shelf-life of an extrudate which comprises vitamin B9 and at least one further water-soluble vitamin preferably selected from the group consisting of vitamin B1 and vitamin B2. Most preferably, the invention relates to the use of a pH buffer to increase the shelf-life of an extrudate which comprises vitamin B9, vitamin B1 and vitamin B2.

Surprisingly, vitamin B9 but also vitamin B1 and vitamin B2 decompose less during storage when the pH buffer of the invention is comprised in extrudates that are obtainable by extruding a composition that comprises vitamin B9, vitamin B1, vitamin B2, at least one filler and water.

The pH buffer as herein described is preferably solid at room temperature and at standard atmosphere. More preferably, the pH buffer as herein described is a mixture of (i) ascorbic acid and (ii) an edible salt of ascorbic acid. Most preferably, the pH buffer as herein described is a mixture of (i) ascorbic acid and (ii) sodium ascorbate. In this embodiment, weight ratio between ascorbic acid and sodium ascorbate is preferably from 1:1 to 1:5, more preferably from 1:1 to 1:4 and most preferably from 1:2.5 to 1:3.5.

Therefore, a preferred embodiment of the invention relates to the use of the pH buffer to increase the shelf-life of an extrudate which comprises vitamin B9, wherein said the pH buffer is a mixture of (i) ascorbic acid and (ii) sodium ascorbate, and wherein the weight ratio between ascorbic acid and sodium ascorbate is preferably from 1:1 to 1:5, more preferably from 1:1 to 1:4 and most preferably from 1:2.5 to 1:3.5.

### Figures

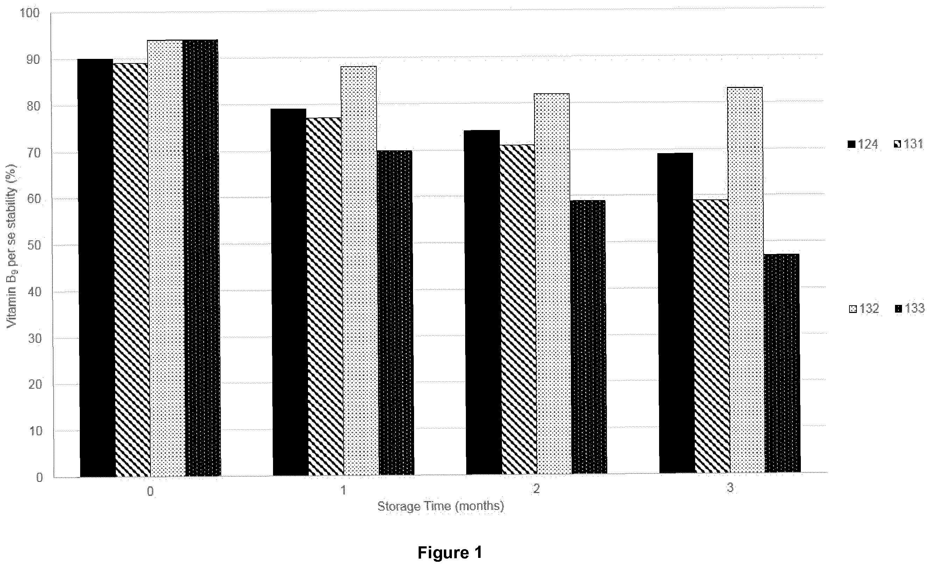

FIGURE 1 shows the result of Example 3. The relative content of vitamin B9 in the extrudates of Example 1 was measured after extrusion (time point: 0 month), and after 1 month, 2 months and 3 months storage time. On the y-axis, the measured vitamin B9 content is indicated in percentage of the calculated vitamin B9 content. Example: 90% vitamin B9 per se stability means that 10% of vitamin B9 which was used to manufacture the extrudates is no longer present.
   Extrudate #132 has higher vitamin B9 per se stability than the other extrudates. Extrudate #132 comprises the pH buffer of the invention.

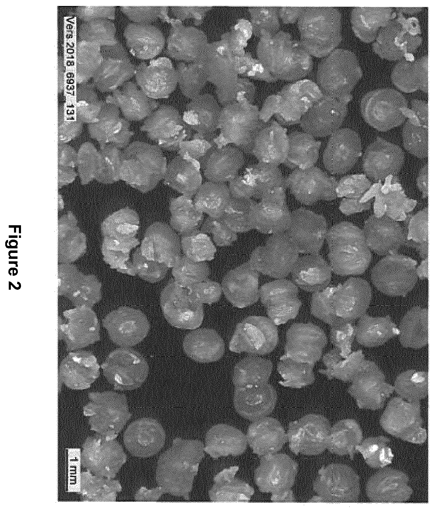

FIGURE 2 shows a plurality of extrudates after drying and without spheronization. The individual extrudates have a diameter between 0.5 mm and 2 mm. The plurality of extrudates resembles a coarse powder and is flowable.

### Examples

### Example 1 (manufacturing of extrudates comprising folic acid)

Extrudates #132 were manufactured as follows:
A powder containing folic acid, a pH buffer (ascorbic Acid and sodium ascorbate), a vitamin mix (vitamins B1, B2, B6, B3 and B12), a filler (wheat starch and durum wheat semolina) and optionally a lubricant was gravimetrically fed (Brabender Technologie) into the first barrel of a laboratory-scale co-rotating twin screw extruder (Thermo Fisher Scientific, HAAKE Polylab OS with PTW16/40 OS twin screw extruder). The extruder consisted of 6 (electrically heated and water-cooled) barrels and a die head (12 or 21 X 0.5 mm) with a screw diameter of 16 mm and a length to diameter ratio of 40. Deionised water was injected into the second barrel. The temperature of barrels 2 - 6 as well as the die head was allowed to reach adiabatic extrusion conditions. The powder premix was fed at 500 g/h and 60 g/h water was added. Strands were then extruded. To obtain a plurality of extrudates, the extruded strands were directly die-face cut by two rotating knives at the die head. After die-face cutting the solid extrudates were dried in a laboratory-scale fluid bed drier (Retsch TG 200) for 60 minutes at 50 °C to obtain a final water content of 6 weight-%, based on the total weight of said extrudates.

Extrudates #133, #131 and #124 were manufactured in the same manner as extrudates #132. The exact composition of all extrudates is shown in below TABLE 1.

**TABLE 1**

| | **Extrudates #132** | **Extrudates #133** | **Extrudates #131** | **Extrudates #124** |
|---|---|---|---|---|
| **ingredient** | weight-% | weight-% | weight-% | weight-% |
| Folic Acid | 0.08 | 0.08 | 0.08 | 0.27 |
| Sodium Ascorbate | 11.35 | - | 15.12 | - |
| Ascorbic Acid | 3.76 | 15.12 | - | - |
| Vitamin mix | 5.02 | 4.73 | 5.02 | 15.73 |
| filler | 73.79 | 74.07 | 73.78 | 77 |
| Lubricant | 6 | 6 | 6 | 7 |

In above TABLE 1, weight-% refers to the total weight of the respective extrudates, not including any residual water. The vitamin mix was identical for all extrudates and comprised thiamine mononitrate (vitamin B1), riboflavin (vitamin B2), pyridoxine hydrochloride (vitamin B6), niacinamide (vitamin B3) and crystalline vitamin B12. The filler and the optional lubricant was also identical for all extrudates.

### Example 2 (pH measurement)

The pH of extrudates #132 was measured as follows:
A plurality of extrudates #132 having a total mass of 1 g was added to 40 ml deionised water at room temperature and allowed to stir for 1h to ensure all extrudates were dispersed. The pH was then measured using a Metrohm 744 pH meter.

The pH of extrudates #133, #131 and #124 was measured in the same manner as the pH of extrudates #132. The result of these measurements is indicated in below TABLE 2.

**TABLE 2**

| **Example** | **Extrudate** | **pH buffer** | **pH** |
|---|---|---|---|
| 1 | #132 | ascorbic acid and sodium ascorbate | 4.73 |
| 2 | #133 | ascorbic acid only | 3.7 |
| 3 | #131 | sodium ascorbate only | 6.25 |
| 5 | #124 | no ascorbic acid | 4.51 |
| | | no sodium ascorbate | |

### Example 3 (storage stability)

The relative content of vitamin B9 in extrudates of Example 1 was measured after extrusion (time point: 0 month), and after 1 month, 2 months and 3 months storage time. Storage conditions: 30°C, 65% humidity, in closed aluminium bags.

The following method was applied:
2 g of the respective extrudates was weighed into a volumetric flask and allowed to dissolve at pH 7 in mobile phase at pH 7. After a set period of time, an aliquot (10 µl) was injected onto a Nucleosil 100-7 C18 (Macherey & Nagel) column connected to an Agilent HPLC system equipped with a UV/VIS detector. Mobile phase consisted of 13.5% Acetonitrile and 86.5% 0.25N pentasulfonate/0.25N heptasulfonate Na and was kept at a constant flow rate of 1.4 ml min⁻¹. Vitamin B9 was detected at a wavelength of 290 nm and the area of the signal was calculated using a standard graph.

The results of the measurement are shown in FIGURE 1. FIGURE 1 shows that less vitamin B9 decomposes during storage if the pH buffer system of the invention had been added to the composition which was extruded.

## Claims

1. Extruded strand, wherein said extruded strand comprises vitamin B9, at least one pH buffer, at least one filler and water.

2. Extruded strand according to claim 1, wherein said pH buffer is solid at room temperature and wherein said pH buffer is preferably a mixture of (i) ascorbic acid and (ii) an edible salt of ascorbic acid.

3. Extruded strand according to claim 1 or 2, wherein said extruded strand comprises at least one further micronutrient and wherein said at least one further micronutrient is preferably selected from the group consisting of minerals, fat-soluble vitamins, water-soluble vitamins, amino acids, polyunsaturated fatty acids and carotenoids.

4. Extruded strand according to any one of claims 1 to 3, wherein said extruded strand comprises at least one further water-soluble vitamin preferably selected from the group consisting of vitamin B1 and vitamin B2.

5. Extruded strand according to any one of claims 1 to 4, wherein said extruded strand comprises from 15 weight-% to 60 weight-% water, preferably from 20 weight-% to 55 weight-% water and more preferably from 25 weight-% to 45 weight-% water, based on the total weight of the extruded strand.

6. A plurality of extrudates, wherein the extrudates are obtainable by cutting the extruded strand according to any one of claims 1 to 5, and wherein said cutting is optionally followed by spheronization.

7. Plurality of extrudates obtainable by drying the extrudates of claim 6, wherein the extrudates comprise preferably less than 10 weight-% water, more preferably less than 8 weight-% water and most preferably less than 5 weight-% water, based on the total weight of said extrudates.

8. Plurality of extrudates according to claim 7, wherein the pH of a liquid obtainable by dispersing 1 g of the extrudates in 40 ml deionised water is at room temperature from 4.6 to 6, preferably from 4.65 to 5.2 and more preferably from 4.7 to 5.

9. Plurality of extrudates according to claim 7 or 8, wherein the vitamin B9 content of the extrudates is after 3 months storage in a closed aluminium bag at 30°C and 65% humidity at least 70%, preferably at least 75% and most preferably at least 80% of the vitamin B9 content of said extrudates before storage.

10. Food, feed or instant powder comprising a plurality of extrudates according to any one of claims 6 to 9, wherein said instant powder is suitable for preparing a beverage.

11. Method of manufacturing a plurality of extrudates, wherein a composition comprising vitamin B9, at least one pH buffer, at least one filler and water is extruded.

12. Method according to claim 11, wherein the temperature at the die of the extruder is from 50°C to 120°C, preferably from 55°C to 90°C and more preferably from 60°C to 80°C.

13. Method according to claim 11 or 12, wherein the die of the extruder has at least one hole, and wherein the diameter of said hole is preferably from 0.1 mm to 2 mm, more preferably from 0.3 mm to 1.2 mm and most preferably from 0.4 mm to 0.8 mm.

14. Use of a pH buffer to increase shelf-life of an extrudate which comprises vitamin B9.

15. Use according to claim 14 or method according to any one of claims 11 to 13, wherein said pH buffer is solid at room temperature and wherein said pH buffer is preferably a mixture of (i) ascorbic acid and (ii) an edible salt of ascorbic acid, and wherein said edible salt of ascorbic acid is preferably sodium ascorbate.

## Patentansprüche

1. Extrudierter Strang, wobei der extrudierte Strang Vitamin B9, mindestens einen pH-Puffer, mindestens einen Füllstoff und Wasser umfasst.

2. Extrudierter Strang nach Anspruch 1, wobei der pH-Puffer bei Raumtemperatur fest ist und wobei es sich bei dem pH-Puffer vorzugsweise um eine Mischung von (i) Ascorbinsäure und (ii) einem essbaren Salz von Ascorbinsäure handelt.

3. Extrudierter Strang nach Anspruch 1 oder 2, wobei der extrudierte Strang mindestens einen weiteren Mikronährstoff umfasst und wobei der mindestens eine weitere Mikronährstoff vorzugsweise aus der Gruppe bestehend aus Mineralien, fettlöslichen Vitaminen, wasserlöslichen Vitaminen, Aminosäuren, mehrfach ungesättigten Fettsäuren und Carotinoiden ausgewählt ist.

4. Extrudierter Strang nach einem der Ansprüche 1 bis 3, wobei der extrudierte Strang mindestens ein weiteres wasserlösliches Vitamin umfasst, das vorzugsweise aus der Gruppe bestehend aus Vitamin B1 und Vitamin B2 ausgewählt ist.

5. Extrudierter Strang nach einem der Ansprüche 1 bis 4, wobei der extrudierte Strang 15 Gew.-% bis 60 Gew.-% Wasser, vorzugsweise 20 Gew.-% bis 55 Gew.-% Wasser und weiter bevorzugt 25 Gew.-% bis 45 Gew.-% Wasser, bezogen auf das Gesamtgewicht des extrudierten Strangs, umfasst.

6. Mehrere Extrudate, wobei die Extrudate durch Schneiden des extrudierten Strangs nach einem der Ansprüche 1 bis 5 erhältlich sind und wobei auf das Schneiden gegebenenfalls eine Sphäronisierung folgt.

7. Mehrere Extrudate, die durch Trocknen der Extrudate nach Anspruch 6 erhältlich sind, wobei die Extrudate vorzugsweise weniger als 10 Gew.-% Wasser, weiter bevorzugt weniger als 8 Gew.-% Wasser und ganz besonders bevorzugt weniger als 5 Gew.-% Wasser, bezogen auf das Gesamtgewicht der Extrudate, umfassen.

8. Mehrere Extrudate nach Anspruch 7, wobei der pH-Wert einer durch Dispergieren von 1 g der Extrudate in 40 ml vollentsalztem Wasser erhältlichen Flüssigkeit bei Raumtemperatur 4,6 bis 6, vorzugsweise 4,65 bis 5,2 und weiter bevorzugt 4,7 bis 5 beträgt.

9. Mehrere Extrudate nach Anspruch 7 oder 8, wobei der Vitamin-B9-Gehalt der Extrudate nach 3 Monaten Lagerung in einem verschlossenen Aluminiumbeutel bei 30 °C und 65 % Feuchte mindestens 70 %, vorzugsweise mindestens 75 % und ganz besonders bevorzugt mindestens 80 % des Vitamin-B9-Gehalts der Extrudate vor der Lagerung beträgt.

10. Nahrungsmittel, Futter oder Instant-Pulver, umfassend mehrere Extrudate nach einem der Ansprüche 6 bis 9, wobei das Instant-Pulver für die Herstellung eines Getränks geeignet ist.

11. Verfahren zur Herstellung von mehreren Extrudaten, bei dem man eine Zusammensetzung, die Vitamin B9, mindestens einen pH-Puffer, mindestens einen Füllstoff und Wasser umfasst, extrudiert.

12. Verfahren nach Anspruch 11, bei dem die Temperatur an der Düse des Extruders 50 °C bis 120 °C, vorzugsweise 55 °C bis 90 °C und weiter bevorzugt 60° Celsius bis 80 °C beträgt.

13. Verfahren nach Anspruch 11 oder 12, bei dem die Düse des Extruders mindestens ein Loch aufweist und wobei der Durchmesser des Lochs vorzugsweise 0,1 mm bis 2 mm, weiter bevorzugt 0,3 mm bis 1,2 mm und ganz besonders bevorzugt 0,4 mm bis 0,8 mm beträgt.

14. Verwendung eines pH-Puffers zur Erhöhung der Haltbarkeit eines Extrudats, das Vitamin B9 umfasst.

15. Verwendung nach Anspruch 14 oder Verfahren nach einem der Ansprüche 11 bis 13, wobei der pH-Puffer bei Raumtemperatur fest ist und wobei es sich bei dem pH-Puffer vorzugsweise um eine Mischung von (i) Ascorbinsäure und (ii) einem essbaren Salz von Ascorbinsäure handelt und wobei es sich bei dem essbaren Salz von Ascorbinsäure vorzugsweise um Natriumascorbat handelt.

## Revendications

1. Brin extrudé, ledit brin extrudé comprenant de la vitamine B9, au moins un tampon de pH, au moins une charge et de l'eau.

2. Brin extrudé selon la revendication 1, ledit tampon de pH étant solide à température ambiante et ledit tampon de pH étant préférablement un mélange (i) d'acide ascorbique et (ii) d'un sel comestible d'acide ascorbique.

3. Brin extrudé selon la revendication 1 ou 2, ledit brin extrudé comprenant au moins un micronutriment supplémentaire et ledit au moins un micronutriment supplémentaire étant préférablement choisi dans le groupe constitué par des minéraux, des vitamines liposolubles, des vitamines hydrosolubles, des acides aminés, des acides gras polyinsaturés et des caroténoides.

4. Brin extrudé selon l'une quelconque des revendications 1 à 3, ledit brin extrudé comprenant au moins une vitamine hydrosoluble supplémentaire choisie dans le groupe constitué par la vitamine B1 et la vitamine B2.

5. Brin extrudé selon l'une quelconque des revendications 1 à 4, ledit brin extrudé comprenant de 15 % en poids à 60 % en poids d'eau, préférablement de 20 % en poids à 55 % en poids d'eau et plus préférablement de 25 % en poids à 45 % en poids d'eau, sur la base du poids total du brin extrudé.

6. Pluralité d'extrudats, les extrudats pouvant être obtenus en découpant le brin extrudé selon l'une quelconque des revendications 1 à 5, et ladite découpe étant éventuellement suivie par une sphéronisation.

7. Pluralité d'extrudats pouvant être obtenue par séchage des extrudats selon la revendication 6, les extrudats comprenant préférablement moins de 10 % en poids d'eau, plus préférablement moins de 8 % en poids d'eau et le plus préférablement moins de 5 % en poids d'eau, sur la base du poids total desdits extrudats.

8. Pluralité d'extrudats selon la revendication 7, le pH d'un liquide pouvant être obtenu en dispersant 1 g des extrudats dans 40 ml d'eau désionisée étant à température ambiante de 4,6 à 6, préférablement de 4,65 à 5,2 et plus préférablement de 4,7 à 5.

9. Pluralité d'extrudats selon la revendication 7 ou 8, la teneur en vitamines B9 des extrudats après 3 mois de stockage dans un sac d'aluminium fermé à 30 °C et 65 % d'humidité étant d'au moins 70 %, préférablement d'au moins 75 % et le plus préférablement d'au moins 80 % de la teneur en vitamines B9 desdits extrudats avant stockage.

10. Produit alimentaire, nourriture ou poudre instantanée comprenant une pluralité d'extrudats selon l'une quelconque des revendications 6 à 9, ladite poudre instantanée étant appropriée pour la préparation d'une boisson.

11. Procédé de fabrication d'une pluralité d'extrudats, une composition comprenant de la vitamine B9, au moins un tampon de pH, au moins une charge et de l'eau étant extrudée.

12. Procédé selon la revendication 11, la température au niveau de la matrice de l'extrudeuse étant de 50 °C à 120 °C, préférablement de 55 °C à 90 °C et plus préférablement de 60 °C à 80 °C.

13. Procédé selon la revendication 11 ou 12, la matrice de l'extrudeuse possédant au moins un trou, et le diamètre dudit trou étant préférablement de 0,1 mm à 2 mm, plus préférablement de 0,3 mm à 1,2 millimètre et le plus préférablement de 0,4 mm à 0,8 mm.

14. Utilisation d'un tampon de pH pour augmenter la durée de conservation d'un extrudat qui comprend de la vitamine B9.

15. Utilisation selon la revendication 14 ou procédé selon l'une quelconque des revendications 11 à 13, ledit tampon de pH étant solide à température ambiante et ledit tampon de pH étant préférablement un mélange (i) d'acide ascorbique et (ii) d'un sel comestible d'acide ascorbique, et ledit sel comestible d'acide ascorbique étant préférablement l'ascorbate de sodium.
